# EUROPEAN PATENT SPECIFICATION

(11) **EP 4 302 857 B1**
(45) Date of publication and mention of the grant of the patent: **30.07.2025**
(21) Application number: 22182894.0
(22) Date of filing: 04.07.2022
(51) Int. Cl.: B01D 46/00, B01D 46/26, B01D 46/69

(54) **FILTERING APPARATUS FOR FILTERING AIR FROM AN ABSORBENT ARTICLES MANUFACTURING PROCESS LINE**
FILTERVORRICHTUNG ZUM FILTERN VON LUFT AUS EINER PRODUKTIONSANLAGE FÜR ABSORBIERENDE ARTIKEL
APPAREIL DE FILTRAGE DE L'AIR À PARTIR D'UNE LIGNE DE TRAITEMENT DE FABRICATION DE LIGNE D'ARTICLES ABSORBANTS

(43) Date of publication of application: 10.01.2024
(73) Proprietor: Ontex BV, 9255 Buggenhout (BE); Ontex Group NV, 9320 Erembodegem (BE)
(72) Inventor: Heege, Thomas, 56761 Düngenheim (DE)
(74) Representative: Saurat, Thibault

(56) References cited:
- US-A- 4 481 021
- US-A- 5 181 945
- US-B1- 8 597 391

## Description

### TECHNICAL FIELD

The present disclosure relates to a filtering apparatus for filtering air from, or coming from, a process line for manufacturing absorbent articles. The present disclosure also pertains to a method of filtering and recycling absorbent material from said process line. The disclosure also relates to the use of such an apparatus for the execution of said method.

### BACKGROUND

A process line for manufacturing absorbent articles, namely disposable hygiene products such as diapers, sanitary napkins or incontinence pants comprises several stations to carry out different steps. Some of these steps require vacuum to ensure the suction or the blowing of components of the absorbent articles, e.g. maintaining absorbent core onto a drum or depositing a cut-out onto a web. Providing vacuum at different stations implies using a sophisticated air circulation system as well as an air filtering system. The manufacturing of absorbent articles implies using absorbent material such as fluff and/or superabsorbent particles, which often get suctioned into the air circulation system and into the air filtering system. Because of this suction of absorbent material, the filtering media often get clogged up and needs to be replaced too often.

Documents US4481021A, US5181945A, US8597391B1 disclose such air filtering systems, yet can be further improved.

The disclosure thereto aims to provide an apparatus and method which ensures an efficient and reliable filtering of the pressurized air present in a process line to manufacture absorbent articles.

### SUMMARY OF THE DISCLOSURE

The present disclosure provides a filtering apparatus for filtering air from an absorbent articles manufacturing process line, said filtering apparatus comprising:
- a housing comprising at least one inlet and preferably defining a sealed inner volume ;
- a filtering media arranged downstream with respect to the airflow of the at least one inlet ;
- a support structure comprising a cylindrical drum comprising a first and second longitudinal end, the filtering media being secured to said cylindrical drum;
- a motor device to rotate the cylindrical drum; and
- a first pump.

According to the disclosure, the motor device comprises a driving mean and the cylindrical drum comprises the corresponding driven mean arranged at the periphery of one of the longitudinal ends of the cylindrical drum, the driving mean actuating the driven mean and cylindrical drum,
and the filtering apparatus comprises a second pump arranged to suck up a lesser portion of the air incoming in the housing than the first pump,
and the filtering apparatus comprises at least one nozzle arranged in proximity of the filtering media and at least one duct linking said nozzle to the second pump,
and the second pump comprise an inlet that is linked to the one or more nozzles and a first and second outlet, the first outlet conveying the air toward the filtering media and the second outlet conveying the air toward said process line.

In other words, the motor device comprises an actuator comprising at least one male or female rotation member, i.e. the driving mean(s), and the cylindrical drum comprises the corresponding, or complementary, female or male rotation member, i.e. the driven mean(s), for driving engagement therewith, the male or female rotation member(s) of the cylindrical drum being arranged at the periphery of one of the longitudinal ends of the cylindrical drum and the male or female rotation member of the actuator actuating the male or female rotation member of the cylindrical drum.

According to an embodiment, the driving mean comprises a geared plate and the driven mean comprises a ring gear, the teeth of the geared plate engaging the teeth of the ring gear.

According to an embodiment, at least one nozzles is arranged between about 0,5 mm and about 4,0 mm distant of the filtering media, preferably between about 0,5 mm and about 3,0 mm, more preferably between about 0,5 and about 2,0 mm, even more preferably between about 0,5 mm and 1,0 mm, distant of the filtering media.

According to an embodiment, two or more nozzles are not aligned with respect to the longitudinal axis of the cylindrical drum.

According to an embodiment, the filtering apparatus comprises a valve to guide the air through the first outlet or through the second outlet.

According to an embodiment, the filtering apparatus comprises a frame comprising a plurality of struts to carry the support structure.

All of these embodiments mentioned above can be taken individually or in combination.

The disclosure also pertains to a method for filtering and recycling absorbent material from an absorbent articles manufacturing process line using a filtering apparatus as described above.

The disclosure also pertains to the use of a filtering apparatus as described above to filter the air coming from a process line and recycle the absorbent material in said air.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG. 1** illustrates a view in perspective of the filtering apparatus according to the disclosure;
**FIG. 2** illustrates a view of a portion of the filtering apparatus, namely the driving mechanism;
**FIG. 3** illustrates a schematic diagram of the filtering apparatus and process line.

### DETAILED DESCRIPTION OF THE DISCLOSURE

The present disclosure concerns an improved apparatus and method for unwinding a web material.

Unless otherwise defined, all terms used in disclosing the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, tampons, diaper holders and liners, pantiliners, sanitary napkins and the like, as well as surgical bandages and sponges. Disposable absorbent articles comprise a liquid pervious topsheet, a liquid impervious backsheet joined to the topsheet, and an absorbent core positioned and held between the topsheet and the backsheet. The absorbent article, or absorbent assembly, may also include other components, such as liquid wicking layers, liquid intake layers, liquid distribution layers, transfer layers, barrier layers, wrapping layers and the like, as well as combinations thereof.

"Absorbent material" as used herein refers to the material constituting the absorbent core of the absorbent article. Examples of commonly occurring absorbent materials are cellulosic fluff pulp, tissue layers, highly absorbent polymers or superabsorbent polymer particles (SAP), absorbent foam materials, absorbent nonwoven materials or the like. It is common to combine cellulosic fluff pulp with superabsorbent polymers in an absorbent material.

The term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp", "cellulosic fluff" or "fluff" refers to a material made up of cellulose fibers. The fibers can be either natural or synthetic, or a combination thereof. The material is typically lightweight and has absorbent properties.

The terms "superabsorbent" or "high absorbency" refer to materials that are capable of absorbing at least 10 times their own weight in liquid. Superabsorbent materials suitable for use in the present disclosure are known to those skilled in the art, and may be in any operative form, such as particulate form, fibers and mixtures thereof. Generally stated, the "superabsorbent material" can be a water-swellable, organic or inorganic, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The terms "downstream" and "upstream" as used herein are with respect to the airflow stream, meaning that the airflow goes from an upstream position to a downstream position.

The terms "air filtering apparatus" and "filtering apparatus" refer to the same apparatus, similarly as "inlet" and "air inlet" reefing to the same inlet.

The terms "height", "bottom", "ceiling", "ground" are all in reference to the apparatus and its components once installed and in a functioning state.

The term "nonwoven fabric" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The recitation of numerical ranges by endpoints includes all numbers and fractions subsumed within that range, as well as the recited endpoints.

In the following detailed description of the preferred embodiments, reference is made to the accompanying drawings that form a part hereof, and in which are shown by way of illustration only of specific embodiments in which the disclosure may be practiced. It is to be understood that other embodiments may be utilised and structural or logical changes may be made without departing from the scope of the present disclosure. The following detailed description, therefore, is not to be taken in a limiting sense, and the scope of the present disclosure is defined by the appended claims.

With regard to these non-limiting examples, the described apparatus and method makes it possible to effectively and efficiently filter air coming from an absorbent article manufacturing process line and to recycle the absorbent material scattered in said process line.

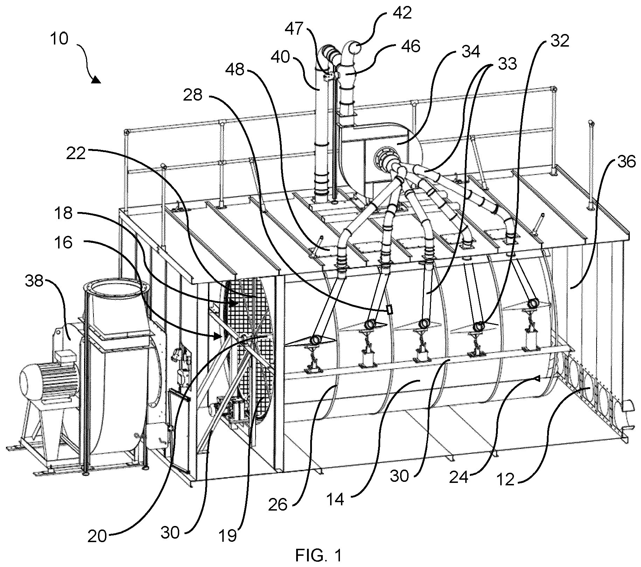

FIG. 1 illustrates a filtering apparatus 10, or air filtering installation, for filtering the air coming from a process line configured to manufacture absorbent articles such as diapers, incontinence or training pants, sanitary napkins, underpads for example, and recycling the absorbent material present in said air. The filtering apparatus 10 for filtering air from an absorbent articles manufacturing process line, said filtering apparatus 10 comprises:
- a housing 36 comprising at least one inlet 12;
- a filtering media 14 arranged downstream with respect to the airflow of the at least one inlet 12;
- a support structure 16 comprising a cylindrical drum 18 comprising a first and second longitudinal end 49,60, the filtering media 14 being secured to said cylindrical drum 18;
- a motor device 52 to rotate the cylindrical drum 18; and
- a first pump 38.

According to the disclosure, the motor device 52 comprises a driving mean 56 and the cylindrical drum 18 comprises the corresponding driven mean 50 arranged at the periphery of one of the longitudinal ends 49,60 of the cylindrical drum 18, the driving mean 56 actuating the driven mean and cylindrical drum 18.

The air filtering apparatus 10 preferably comprises a plurality of inlets 12 or air inlets, for example five air inlets 12 as illustrated in FIG.1. The disclosure is not limited to this number of inlets 12. The filtering apparatus 10 may comprise two, three, four, six, seven, eight, nine, ten or more inlets 12.

The filtering apparatus 10 comprises a support structure 16 to carry, support or maintain, the filtering media 14 in a steady position, the filtering media 14. For example, the support structure 16 comprises a cylindrical drum 18 elongated, or extending, in a longitudinal direction and comprising a grid 19, latticework or mesh 19 or in other words a weblike pattern or construction 19 that can retain the filtering media 14 while being air-permeable. The mesh 19 defines the lateral surface of the cylindrical drum 18. The cylindrical drum 18 can further comprise a central shaft 20 around which the cylindrical drum 18 rotates and optionally several reinforcements 22 such as rods, or arms, linking the central shaft 20 to the cylindrical drum 18 or bridging two diametrically opposed points of the cylindrical drum 18. The reinforcements 22 can extend in the radial direction and/or in the longitudinal direction of the cylindrical drum 18. The filtering media 14 is fastened, or secured, onto the support structure 16. The filtering media 14 can for example comprise a zipper system 24 comprising a slider connecting two transversal ends of the filtering media 14 around the cylindrical drum 18, the transversal ends comprising two rows of teeth that are designed to interlock thereby joining the transversal ends together around the cylindrical drum 18. The filtering media 14 can also be fastened onto the cylindrical drum 18 with straps 26 and corresponding buckles 28. As illustrated in FIG. 1, the air filtering apparatus 10 comprises here five straps 26 to further secure the filtering media 14 to the cylindrical drum 18 in addition of the zipper 24.

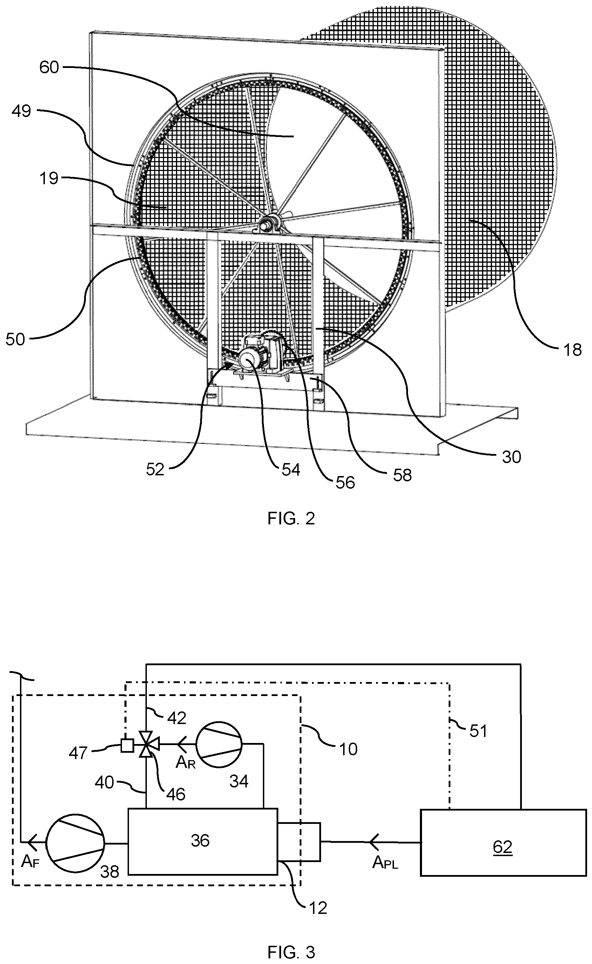

The central shaft 20 when present can extend along the entire length of the cylindrical drum 18, or as illustrated in FIG. 2, the central shaft can extend along a portion of the longitudinal axis of the cylindrical drum 18.

The filtering apparatus 10 may also comprise a frame 30 to maintain the support structure 16. The frame 30, or space frame, comprises a plurality of struts and/or reinforcements such as rods or arms forming a truss-like structure to carry and support the support structure 16, in particular the cylindrical drum 18.

The air filtering apparatus 10 comprises at least one nozzle 32, for example, two, three, four, five, six, seven, eight, nine, ten or more nozzles 32, here five nozzles are illustrated in FIG.1. The one or more nozzles 32 are linked through ducts 33 to a pump 34, that will be referenced as the recycling pump 34 henceforth, enabling a suction of air and absorbent material contained in said air. The plurality of nozzles 32 are arranged in close proximity to the filtering media 14. More precisely, the recycling pump 34 is arranged and configured to suck up a portion, meaning not the entirety, of the air incoming in the housing 36.

Each nozzle 32 comprises an inlet which is preferably convergent with respect to the airflow direction where the cross section of the nozzle is narrowing down, or decreasing, from a greater cross section to a lesser cross section in the direction of the flow. By being convergent and in close proximity of the filtering media 14, the surface of suction of the nozzle 32 is greater thereby increasing the amount of absorbent material to be suctioned by the recycling pump 34. In other words, the greater cross section 32 of the nozzle is closest to the filtering media 14 whereas the lesser cross section of the nozzle 32 is closest to the duct 33 with respect to the airflow direction.

The air filtering apparatus 10 also comprises a housing 36 defining an inner volume with a set of walls, for example four walls and a ground wall and ceiling wall and optionally partition walls within said inner volume. The filtering media 14, support structure 16, nozzles 32 and frame 30 are arranged at least partially within said inner volume. The housing 36 enables to contain the air to be filtered within said inner volume and directs the airflow to pass through the filtering media 14. The air filtering apparatus 10 also comprises a pump 38, that will be referenced as the main pump 38 henceforth, enabling a suction of air and absorbent material contained within said air. The main pump 38 is preferably arranged downstream of the filtering media 14 with respect to the airflow. The main pump 38 comprises an inlet that is in fluidic connection with the inner volume defined by the housing 36 and an outlet that is arranged outside, or in the exterior of, the housing 36, or at least in fluidic connection with the exterior of the housing.

The main pump 38 and the recycling pump 34 are both means for moving fluids, in this case air. The main pump 38 can be referenced as the first pump 38 and the recycling pump 34 can be reference as the second pump 38. The present disclosure is not limited to the nature of these pumps. They can be selected from and not limited to centrifugal pumps such as centrifugal fans, axial-flow pumps, diaphragm pumps, impeller pumps, screw pumps, booster pumps, canned motor pump, circulator pumps, single or multistage pumps and turbine pumps. The main pump 38 is the pump ensuring the vacuum in the absorbent articles manufacturing process line and has preferably a higher volume flow rate, or air capacity, then the recycling pump 34. In other words, the main pump 38 enables a greater flow than the recycling pump 34, *i.e.* the flow of air passing through the main pump 38 is greater than the flow passing through the recycling pump 34. The volume flow rate, or air capacity, of the main pump 38 is at least ten times greater than the air capacity of the recycling pump 34. Preferably, the volume flow rate, or air capacity, of the main pump 38 is at least twenty times, more preferably between 25 and 50 times greater than the air capacity of the recycling pump 34. For example, the recycling pump 34 has a volume flow rate of about 1000 m³/h whereas the main pump 38 has a volume flow rate between about 30 000 m³/h and about 40 000 m³/h. The main pump 38 and the recycling pump 34 are preferably of the same nature, for example, the main pump 38 and the recycling pump 34 are both centrifugal fans. Alternatively the main pump 38 and the recycling pump 34 can be of different nature. For instance, given that the main pump 38 is destined to move air and the recycling pump 34 is destined to move air and absorbent material, the recycling pump can have specific seals and a impeller adapted to avoid clogging. Given that the main pump 38 has a greater air capacity than the recycling pump 34, the main pump 38 will suck in the majority of the air incoming in the housing 36 and the recycling pump 34, through the nozzle will suck a lesser portion of that air than the main pump 38. In other words, given the arrangement and the air capacities of the pumps 34,38, the majority of the air incoming in the housing 36 flows through the main pump 38 and a minority of that incoming air flows through the recycling pump 34.

Following the airflow, the air coming from the process line and containing absorbent material such as cellulosic fibers e.g. fluff and/or superabsorbent particles, enters the filtering apparatus 10, namely enters within the inner volume defined by the housing 36 through the one or more air inlets 12. The air then passes through the filtering media 14 and is then extracted from the inner volume by the main pump 38. The filtering media 14 filters the air so that the absorbent material (cellulosic fibers and/or SAP) contained in the air is retained within, or on the surface of, the filtering media 14.

The one or more nozzles 32 being placed in close proximity to the filtering media 14 enable to extract said absorbent material from the filtering media 14. The recycling pump 34 comprises one inlet that is linked one or more nozzles 32 and a first and second outlets 40,42. The first outlet 40 is in fluidic connection with the inner volume defined by the housing 36. In other words, the recycling pump 38 can re-inject the air and absorbent material within the air filtering apparatus 10 and ensure a re-filtering of the air. The second outlet 42 is in fluidic connection with the process line, namely with the absorbent core forming part of the process line where the recycled absorbent material can be re-used and arranged within an absorbent article. The recycling pump 34 or outlet duct of said pump 34 preferably comprises a valve 46 to guide the air through one outlet or the other. When the process line for manufacturing absorbent articles is running, the valve 46 guides the air and absorbent material towards the second outlet 42 to recycle the absorbent material whereas if the process line is temporarily stopped, the valve 46 guides the air and absorbent material toward the first outlet 40 to refilter the air and ensure a closed air loop. The valve 46 is preferably a three-port valve such as a three-way ball valves come with a L- shaped fluid passageways inside the rotor. The valve 46 also comprises an actuator 47 to control the direction of the airflow, preferably the valve 46 comprises an electromechanical actuators such as an electric motor or solenoid to enable an automatic control or a remote control. For instance, when the process line is temporarily stopped, it can send, or emit, an electronic signal 51 to the actuator 47 to orientate the airflow towards the first outlet 40 and housing 36.

The airflow and apparatus 10 are schematically illustrated in FIG. 3. The air A_{PL} coming from the process line 62 in which absorbent articles are manufactured is conveyed towards filtering apparatus 10 and enters the inner volume of the housing via the inlet(s) 12, here via two inlets. At this point, the air A_{PL} contains an average amount of absorbent material. The air is then filtered, air A_{F}, and is conveyed towards the exterior by the main pump 38. This filtered air A_{F} contains none or an infinitesimal amount of absorbent material. Alternatively, the recycled absorbent material is suctioned by the nozzles and recycling pump 34 and the recycled air A_{R} is either sent back to the absorbent articles manufacturing process line 62 via the second outlet 42 or sent back to the filtering media 14 via the first outlet 41. The recycled absorbent material-laden air A_{R} contains an average or greater amount of absorbent material.

The housing 36 can comprise one or more transparent, or see-through, panels 48 to avoid installing electric lighting in the housing 36.

In order to ensure an efficient filtering of the air and recycling of the absorbent material, it is preferable that the air filtering apparatus 10 comprises an actuation mean for the filtering media 14 so that the nozzles 32 can extract the absorbent material on the entire surface of the filtering media 14 and not just on specific portions. Actuating the nozzles 32 is undesired as it would be more complicated because of the ducts 33 and the frame 30.

The filtering apparatus 10 comprises a motor device 52 to actuate the support structure 16, namely the cylindrical drum 18. The air filtering apparatus 10 may comprise a motor directly engaging the central saft 20 and inducing a rotary motion on the shaft 20 and thereby on the cylindrical drum 18 given the reinforcements 22 linking the central shaft 20 to the cylindrical drum 18. While simple to implement, there is a significant loss of torque as the driving force is applied on the central shaft 20 meaning at a distance from the main body of the cylindrical drum 18. According to an embodiment of the present disclosure, the filtering apparatus 10 comprises a motor device 52 directly engaging the cylindrical drum 18 and inducing a rotary motion directly on the cylindrical drum 18 and not at a distance.

For this, the filtering device 10 comprises a driving mean and a driven mean engaging one another and arranged at the periphery of the cylindrical drum 18. As illustrated in FIG. 2, the cylindrical drum 18 comprises at one of its longitudinal ends a ring gear 50, or annular gear, with the teeth being extending radially inwards or radially outwards of the ring gear 50, here inwards. The air filtering apparatus 10 comprises a motor device 52 to actuate the ring gear 50 and induce a rotary motion to the cylindrical drum 18. The motor device 52 comprises a motor 54, such as an electric motor, driving in rotation, for example with a shaft, a geared plate 56, or pinion, which is engaging the teeth of the ring gear 50. The motor 54 actuates the plate 56 which in turns transmit this rotary motion to the ring gear 50 and cylindrical drum 18. The torque and power supplied by the motor 54 is directly transmitted to the main body of the cylindrical drum 18. The disclosure is not limited to this type of transmission. For instance the motor device can be selected from and not limited to a spur gear system, a worm drive, a belt transmission or an epicyclic gearing or planetary gearing. Similarly, the disclosure is not limited to these shapes, the gears can be selected from and not limited to spur gears, bevel gears, crown gears, spiral bevel gears, helical gears or herringbone gears. The present disclosure encompasses embodiments where elements such as gaskets are arranged between the cylindrical drum 18 and the ring gear 50. In other words, when saying that the motor device 52 is directly engaging the cylindrical drum 18 and inducing a rotary motion directly on the cylindrical drum 18, it encompasses embodiments where additional elements such as gaskets or additional gears are arranged between the motor device 52 and the cylindrical drum 18. With such an arrangement, the torque of the motor device 52 is directly transmitted to the cylindrical drum 18, more precisely to the main body and lateral face of the cylindrical drum 18, thereby reducing torque loss and the cylindrical drum 18 can be rotated with more power. In other words, by connecting the motor device 52 directly to the cylindrical drum 18, the needed torque to induce a rotation of said cylindrical drum 18 is minimized. Another way to say it, the torque is used or applied where it's really needed meaning on the outer surface of the cylindrical drum 18, i.e. a direct force flow. The geared plate 56 being stiff and stable, it makes the transfer of torque and inducing a rotation of said drum more precise.

Given that the cylindrical drum 18 can be rotated with more power, it is possible to approach the nozzle(s) 32 closer to the surface of the filtering media 14. Conventional nozzles are usually placed about 5,0 mm distant of the filtering media's 14 surface, but because of the better torque and driving force of the motor device 52 onto the cylindrical drum 18, it is possible to arrange the nozzles 32 between about 0,5 mm to about 4,0 mm remote from the filtering media's 18 surface, preferably between about 0,5 mm and about 3,0 mm, more preferably between about 0,5 and about 2,0 mm, even more preferably between about 0,5 mm and 1,0 mm, distant of the filtering media 14. The vacuum, or suction, induced by recycling pump 34 will not affect the rotation of the cylindrical drum 18 at these distances.

With the nozzles 32 being placed closer of the filtering media 14, the absorbent material is thereby extracted and recycled more efficiently. This ensures not only saving in raw material, but also increases the longevity of the filtering media 14 as it gets clogged less often.

As illustrated in FIG. 1, the nozzles 32 are not arranged at the same height, with respect to the cylindrical drum 18 in a functioning, or filtering, position. In other words, two or more nozzles 32 are not aligned with respect to the longitudinal axis of the cylindrical drum 18 *i.e.* with respect to the axis of the central shaft 20. The nozzles 32 and the vacuum induced by the recycling pump 34 is thereby spread out, meaning not at one single rotational position, or not at one vertical position, which has a lesser impact on the rotational movement of the cylindrical drum 18. The nozzles 32 can also be brought closer to the filtering media 14, between about 0,5 mm to 4,0 mm, preferably between 0,5 mm and 1,0 mm thanks to this arrangement of nozzles 32 having a lesser impact on the rotation of the drum 18. Furthermore, absorbent material arrives to the air filtering apparatus in different amounts at different times, for example if there's a malfunction in the process line. Having the nozzles arranged at different height enables to better handle the different arrivals of absorbent material, as it will not reach the same longitudinal portions of the cylindrical drum 18.

As illustrated in FIG. 1, it is preferable that the nozzles are arranged on a same side or half of the cylindrical drum, this is mainly to simplify the ducts 33 and pump 34 arrangement.

As illustrated in FIG. 2, the motor device 52 is elevated by a platform 58 for example here a plate that is bolted to the frame 30 or its reinforcement struts. The cylindrical drum 18, being actuated in rotation, is preferably elevated and maintained above the ground, by the frame 30 structure, hence the motor device 52 actuating said cylindrical drum 18 is also preferably elevated.

The cylindrical drum 18 comprises a first 49 and second 60 longitudinal ends. At the first longitudinal end 49, the cylindrical drum 18 comprises a mean that can be driven or actuated, in this case a ring gear 50 that it can be actuated in rotation by the motor device 52. In other words, the first longitudinal end 49 comprises a mean enabling the cylindrical drum 18 to be rotated. At the opposite longitudinal end, meaning the second longitudinal end 60, the cylindrical drum 18 is preferably closed-off, meaning that an airflow cannot pass through the second longitudinal end 60. The second longitudinal end 60 comprises an air-impermeable mean such as a metal plate preventing the air from passing through. The air entering through the inlets 12 is deflected by the plate and is forced to flow through the filtering media 14 and mesh grid 19. In this embodiment, the filtering media 14 is in the shape or form of a rectangular mat of filtering material that is arranged around the cylindrical drum 18 and is secured or fastened to said drum 18 by the zipper 24 and/or straps 26. In other words, the cylindrical drum 18 is substantially hollow, not taking into account the reinforcements 22 and central shaft 20, and comprises two longitudinal ends defining the bases of the cylinder, one of which is sealed, and a mesh or grid 19 defining the lateral face of the cylinder.

The disclosure is not limited to this embodiment, for instance, the second longitudinal end 60 can also comprise a mesh or grid and the filtering media 14 is in the shape or form of a sleeve of filtering material that is slid onto the cylindrical drum 18.

The one or more inlets 12 are preferably arranged in the bottom, with respect to height, of the housing 36. The air coming from the process line contains absorbent material and that absorbent material can accumulate on the floor on the housing 36. By arranging the inlet(s) 12 in the bottom, the incoming air can pick up some absorbent material that has accumulated on the floor and convey it to the filtering media 14 to be reinjected in the process line *via* the recycling pump 34.

The filtering media 14 is a media filter using a bed of fibrous or porous material such as textile fabric or foam and removes the solid particulate such as the absorbent material from the air. The filtering media 14 can for example be composed of paper, foam, woven or nonwoven fabric or cotton.

The embodiments described above can be taken alone or in combination. For example, the apparatus 10 can comprise three inlets 12 and two straps 26 and a three-port valve 46.

## Claims

1. Filtering apparatus (10) for filtering air from an absorbent articles manufacturing process line, said filtering apparatus (10) comprising
a. a housing (36) comprising at least one inlet (12);
b. a filtering media (14) arranged downstream with respect to the airflow of the at least one inlet (12);
c. a support structure (16) comprising a cylindrical drum (18) comprising a first and second longitudinal end (49,60), the filtering media (14) being secured to said cylindrical drum (18);
d. a motor device (52) to rotate the cylindrical drum (18); and
e. a first pump (38);
wherein the motor device (52) comprises a driving mean (56) and the cylindrical drum (18) comprises the corresponding driven mean (50) arranged at the periphery of one of the longitudinal ends (49,60) of the cylindrical drum (18), the driving mean (56) actuating the driven mean and cylindrical drum (18),
wherein the filtering apparatus (10) comprises a second pump (34) arranged to suck up a lesser portion of the air incoming in the housing (36) than the first pump (38),
wherein the filtering apparatus comprises at least one nozzle (32) arranged in proximity of the filtering media (14) and at least one duct (33) linking said nozzle (32) to the second pump (34),
**characterized in that** the second pump (34) comprise an inlet that is linked to the one or more nozzles (32) and a first and second outlet (40,42), the first outlet (40) conveying the air toward the filtering media (14) and the second outlet (42) conveying the air toward said process line.

2. Filtering apparatus (10) according to claim 1, wherein the driving mean (56) comprises a geared plate (56) and the driven mean (50) comprises a ring gear (50), the teeth of the geared plate (56) engaging the teeth of the ring gear (50).

3. Filtering apparatus (10) according to claim 1 or 2, wherein said at least one nozzles (32) is arranged between about 0,5 mm and about 4,0 mm distant of the filtering media (14), preferably between about 0,5 mm and about 3,0 mm, more preferably between about 0,5 and about 2,0 mm, even more preferably between about 0,5 mm and 1,0 mm, distant of the filtering media (14).

4. Filtering apparatus (10) according to any of the preceding claims, wherein two or more nozzles (32) are not aligned with respect to the longitudinal axis of the cylindrical drum (18).

5. Filtering apparatus (10) according to any of the preceding claims, wherein the filtering apparatus (10) comprises a valve (46) to guide the air through the first outlet (40) or through the second outlet (42).

6. Filtering apparatus (10) according to any of the preceding claims, wherein the volume flow rate of the first pump (38) is at least ten times greater than the volume flow rate of the second pump (34).

7. Filtering apparatus (10) according to any of the preceding claims, wherein the housing (34) comprises four or five air inlets (12).

8. Filtering apparatus (10) according to any of the preceding claims, wherein the housing (34) comprises at least one see-through panels (48) preferably arranged on the ceiling of the housing (36).

9. Filtering apparatus (10) according to any of the preceding claims, wherein the filtering apparatus (10) comprises a frame (30) comprising a plurality of struts to carry the support structure (16).

10. Method for filtering and recycling absorbent material from an absorbent article manufacturing process line using a filtering apparatus (10) according to claim 1 to 9.

11. Use of a filtering apparatus (10) as according to claim 1 to 9 to filter the air coming from a process line and recycle the absorbent material in said air.

## Patentansprüche

1. Filtereinrichtung (10) zum Filtern von Luft aus einer Prozesslinie für Herstellung absorbierender Artikel, die Filtereinrichtung (10) umfassend
a. ein Gehäuse (36), umfassend mindestens einen Einlass (12);
b. ein Filtermedium (14), das hinsichtlich des Luftstroms nachgeschaltet des mindestens einen Einlasses (12) angeordnet ist;
c. eine Trägerstruktur (16), umfassend eine zylindrische Trommel (18), umfassend ein erstes und ein zweites Längsende (49, 60), wobei das Filtermedium (14) an der zylindrischen Trommel (18) gesichert ist;
d. eine Motorvorrichtung (52), um die zylindrische Trommel (18) zu drehen; und
e. eine erste Pumpe (38);
wobei die Motorvorrichtung (52) ein Antriebsmittel (56) umfasst und die zylindrische Trommel (18) das entsprechende angetriebene Mittel (50) umfasst, das an dem Umfang eines der Längsenden (49, 60) der zylindrischen Trommel (18) angeordnet ist, wobei das Antriebsmittel (56) das angetriebene Mittel und die zylindrische Trommel (18) in Bewegung versetzt,
wobei die Filtereinrichtung (10) eine zweite Pumpe (34), die angeordnet ist, um einen geringeren Anteil der in das Gehäuse (36) eintretenden Luft als die erste Pumpe (38) anzusaugen, umfasst,
wobei die Filtereinrichtung mindestens eine Düse (32), die nahe dem Filtermedium (14) angeordnet ist und mindestens eine Leitung (33), die die Düse (32) mit der zweiten Pumpe (34) verknüpft, umfasst,
**dadurch gekennzeichnet, dass** die zweite Pumpe (34) einen Einlass, der mit der einen oder den mehreren Düsen (32) verknüpft ist, und einen ersten und einen zweiten Auslass (40, 42) umfasst, wobei der erste Auslass (40) die Luft zu dem Filtermedium (14) transportiert und der zweite Auslass (42) die Luft zu der Prozesslinie transportiert.

2. Filtereinrichtung (10) nach Anspruch 1, wobei das Antriebsmittel (56) eine verzahnte Platte (56) und das angetriebene Mittel (50) ein Hohlrad (50) umfasst, wobei die Zähne der verzahnten Platte (56) in die Zähne des Hohlrads (50) eingreifen.

3. Filtereinrichtung (10) nach Anspruch 1 oder 2, wobei die mindestens eine Düse (32) zwischen etwa 0,5 mm und etwa 4,0 mm von dem Filtermedium (14) entfernt, vorzugsweise zwischen etwa 0,5 mm und etwa 3,0 mm, mehr bevorzugt zwischen etwa 0,5 und etwa 2,0 mm, noch mehr bevorzugt zwischen etwa 0,5 mm und 1,0 mm, von dem Filtermedium (14) entfernt angeordnet ist.

4. Filtereinrichtung (10) nach einem der vorstehenden Ansprüche, wobei zwei oder mehr Düsen (32) hinsichtlich der Längsachse der zylindrischen Trommel (18) nicht ausgerichtet sind.

5. Filtereinrichtung (10) nach einem der vorstehenden Ansprüche, wobei die Filtereinrichtung (10) ein Ventil (46), um die Luft durch den ersten Auslass (40) oder durch den zweiten Auslass (42) zu führen, umfasst.

6. Filtereinrichtung (10) nach einem der vorstehenden Ansprüche, wobei der Volumendurchfluss der ersten Pumpe (38) mindestens zehnmal größer als der Volumendurchfluss der zweiten Pumpe (34) ist.

7. Filtereinrichtung (10) nach einem der vorstehenden Ansprüche, wobei das Gehäuse (34) vier oder fünf Lufteinlässe (12) umfasst.

8. Filtereinrichtung (10) nach einem der vorstehenden Ansprüche, wobei das Gehäuse (34) mindestens eine durchsichtige Wand (48), die vorzugsweise an der Decke des Gehäuses (36) angeordnet ist, umfasst.

9. Filtereinrichtung (10) nach einem der vorstehenden Ansprüche, wobei die Filtereinrichtung (10) einen Rahmen (30) umfassend eine Vielzahl von Streben, um die Trägerstruktur (16) zu stützen, umfasst.

10. Verfahren zum Filtern und Recyceln von absorbierendem Material aus einer Prozesslinie für Herstellung absorbierender Artikel unter Verwendung einer Filtereinrichtung (10) nach Anspruch 1 bis 9.

11. Verwendung einer Filtereinrichtung (10) nach Anspruch 1 bis 9, um die Luft, die aus einer Prozesslinie kommt, zu filtern und das absorbierende Material in der Luft zu recyceln.

## Revendications

1. Appareil de filtrage (10) permettant de filtrer l'air provenant d'une ligne de traitement de fabrication d'articles absorbants, ledit appareil de filtrage (10) comprenant
a. un boîtier (36) comprenant au moins une entrée (12) ;
b. un média filtrant (14) agencé en aval par rapport au flux d'air de l'au moins une entrée (12) ;
c. une structure de support (16) comprenant un tambour cylindrique (18) comprenant une première et une seconde extrémité longitudinale (49, 60), le média filtrant (14) étant fixé audit tambour cylindrique (18) ;
d. un dispositif moteur (52) pour faire tourner le tambour cylindrique (18) ; et
e. une première pompe (38) ;
dans lequel le dispositif moteur (52) comprend un moyen d'entraînement (56) et le tambour cylindrique (18) comprend le moyen entraîné correspondant (50) agencé à la périphérie de l'une des extrémités longitudinales (49, 60) du tambour cylindrique (18), le moyen d'entraînement (56) actionnant le moyen entraîné et le tambour cylindrique (18),
dans lequel l'appareil de filtrage (10) comprend une seconde pompe (34) agencée pour aspirer une partie moins importante de l'air entrant dans le boîtier (36) que la première pompe (38),
dans lequel l'appareil de filtrage comprend au moins une buse (32) agencée à proximité du média filtrant (14) et au moins un conduit (33) reliant ladite buse (32) à la seconde pompe (34),
**caractérisé en ce que** la seconde pompe (34) comprend une entrée reliée à la ou aux buses (32) et une première et une seconde sortie (40, 42), la première sortie (40) acheminant l'air vers le média filtrant (14) et la seconde sortie (42) acheminant l'air vers ladite ligne de traitement.

2. Appareil de filtrage (10) selon la revendication 1, dans lequel le moyen d'entraînement (56) comprend une plaque dentée (56) et le moyen entraîné (50) comprend une couronne dentée (50), les dents de la plaque dentée (56) venant en prise avec les dents de la couronne dentée (50).

3. Appareil de filtrage (10) selon la revendication 1 ou 2, dans lequel ladite au moins une buse (32) est disposée entre environ 0,5 mm et environ 4,0 mm de distance du média filtrant (14), de préférence entre environ 0,5 mm et environ 3,0 mm, plus préférablement entre environ 0,5 et environ 2,0 mm, encore plus préférablement entre environ 0,5 mm et 1,0 mm, de distance du média filtrant (14).

4. Appareil de filtrage (10) selon l'une quelconque des revendications précédentes, dans lequel deux buses ou plus (32) ne sont pas alignées par rapport à l'axe longitudinal du tambour cylindrique (18).

5. Appareil de filtrage (10) selon l'une quelconque des revendications précédentes, dans lequel l'appareil de filtrage (10) comprend une vanne (46) pour guider l'air à travers la première sortie (40) ou à travers la seconde sortie (42).

6. Appareil de filtrage (10) selon l'une quelconque des revendications précédentes, dans lequel le débit volumétrique de la première pompe (38) est au moins dix fois supérieur au débit volumétrique de la seconde pompe (34).

7. Appareil de filtrage (10) selon l'une quelconque des revendications précédentes, dans lequel le boîtier (34) comprend quatre ou cinq entrées d'air (12).

8. Appareil de filtrage (10) selon l'une quelconque des revendications précédentes, dans lequel le boîtier (34) comprend au moins un panneau transparent (48) agencé de préférence sur le plafond du boîtier (36).

9. Appareil de filtrage (10) selon l'une quelconque des revendications précédentes, dans lequel l'appareil de filtrage (10) comprend un cadre (30) comprenant une pluralité d'entretoises pour porter la structure de support (16).

10. Procédé de filtrage et de recyclage de matériaux absorbants provenant d'une ligne de traitement de fabrication d'articles absorbants à l'aide d'un appareil de filtrage (10) selon les revendications 1 à 9.

11. Utilisation d'un appareil de filtrage (10) selon les revendications 1 à 9 pour filtrer l'air provenant d'une ligne de traitement et recycler le matériau absorbant dans ledit air.
